# EUROPEAN PATENT SPECIFICATION

(11) **EP 0 385 601 B1**
(45) Date of publication and mention of the grant of the patent: **14.06.1995**
(21) Application number: 90301442.1
(22) Date of filing: 12.02.1990
(51) Int. Cl.: A61K 47/48, A61K 49/00, A61K 51/10

(54) **Cross-linked antibodies and processes for their preparation**
Vernetzte Antikörper und Verfahren zu ihrer Herstellung
Anticorps réticulés et leurs procédés de préparation

(30) Priority: 10.02.1989 GB 8903021
(43) Date of publication of application: 05.09.1990
(73) Proprietor: CELLTECH THERAPEUTICS LIMITED, Slough, Berkshire SL1 4EN (GB)
(72) Inventor: Rhind, Stephen Keith, Wooburn, Buckinghamshire, HP10 0QQ (GB)
(74) Representative: Hallybone, Huw George

(56) References cited:
- EP-A- 0 224 120
- WO-A-87/05030
- WO-A-89/01974

## Description

### Field of the Invention

This invention relates to cross-linked antibodies, to compositions containing them, to processes for their preparation, and to their use in medicine and for diagnosis.

### Background to the Invention

Antibody conjugates, in which antibodies are covalently attached to reporter or effector groups, have been used in diagnosis, and, to a more limited extent, in therapy. The antibody is generally selected on the basis of its affinity and specificity for a particular target antigen, such that, in use, it should be able to deliver the reporter of effector group to a desired site and maintain it there for a length of time.

Bispecific heterodimeric antibodies have been previously described in which Fab' fragments have been joined via a thioether linkage [Glennie M.J. et al J. Immunol. 139, 2367, (1987)]. Antibodies in which the fluorescein derivative crabescein has been linked across a disulphide bond have also been described [Packard, B.P. et al, Biochemistry 25, 3548 (1986)]. The labelling of whole antibodies is also described in European Patent Application EP-A-0224120.

We have now found that by cross-linking at least two chains of an antibody fragment in a cross-linkage away from the antigen binding domains the binding capacity of the modified antibody fragment may be advantageously enhanced relative to the unmodified antibody. In vivo, modified antibody fragments of this type also have good blood clearance and, in the presence of a tumour, give advantageously high tumours; blood and tumour: bone ratios. Such antibody fragments, when labelled with a reporter or effector group thus offer potential advantages over conventional labelled antibodies .

### Summary of the Invention

Thus, according to one aspect of the invention we provide an antibody fragment conjugate comprising an antibody fragment specific for one antigenic determinant having covalently attached thereto a reporter or effector group characterised in that the antibody fragment conjugate is cross-linked with at least one non-disulphide interchain bridge, said bridge being attached to each chain at one or more bridging sites located outside of the antigen binding domains of the antibody.

In the antibody fragment conjugates according to the invention, the interchain bridge may link any heavy or light chain in the antibody fragment molecule to one or more other heavy and/or light chains in the same molecule. Preferably, however, the bridge will link two chains, particularly two heavy chains. More than one interchain bridge group may be present, although conjugates with one such bridge are preferred.

The term non-disulphide interchain bridge is intended to mean that S-S bridges of the type normally found in antibodies are excluded.

The bridging site in each antibody fragment chain may generally be at the side-chain of an amino acid residue forming part of the chain but not directly participating in the antigen binding domain. Suitable amino acids include those with a side-chain containing an amino, sulphydryl, carboxyl, phenolic or other aromatic or heteroaromatlc functional group through which the interchain bridge may be linked. Particular amino acid residues of these types include lysine, cysteine, glutamic acid, aspartic acid and tyrosine residues. Alternatively, the bridging site may be at a carbohydrate residue attached to the antibody fragment chain, particularly an oxidised carbohydrate residue containing at least one aldehyde group through which the interchain bridge may be linked.

Particularly preferred bridging sites are the sulphydryl groups of cysteine residues, for example those normally functioning in interchain disulphide bridges. Preferred sites of this type are sulphydryl groups of cysteine residues present in heavy chains in the hinge region of the antibody fragment.

In another preference, the bridging site may be at the side-chain of an amino acid residue not normally present in the immunoglobulin, but which has been introduced through the use of recombinant DNA techniques as described hereinafter. Such sites include the sulphydryl and amino groups of cysteine and lysine residues respectively.

The interchain bridges in conjugates according to the invention may in general be of any desired length or composition. Suitable bridges include residues of homo- or heterofunctional cross-linking reagents, particularly homo- or heterobifunctional cross-linking reagents. Thus, the bridges may be such that they link two or more bridging sites. Particular bridges include optionally substituted polyvalent, especially bivalent, radicals of aliphatic, aromatic or araliphatic compounds.

The interchain bridge may for example have the structure 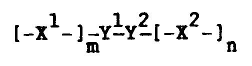
(where X¹ and X² is each the residue of a reactive functional group, Y¹ and Y² together form the remainder of the bridge and m and n, which may be the same or different is each an integer 1 or more).

In bridges of the above particular types Y¹ and Y² together may be straight or branched C₁₋₂₀alkylene (e.g. C₁₋₁₀alkylene such as C₄₋₁₀alkylene, e.g. butylene, pentylene, hexylene, or heptylene), C₂₋₂₀alkenylene or C₂₋₂₀alkynylene chains, optionally interrupted by one or more -O- or -S- atoms or
C₅₋₈cycloalkylene (e.g. cyclopentylene or cyclohexylene), C₆₋₁₂ aromatic (e.g. phenylene or substituted phenylene),
C₅₋₁₀heteroaromatic (e.g. furanyl, pyridyl), -N(R¹)- (where R¹ is a hydrogen atom or a C₁₋₆alkyl group), -CON(R¹)- or -N(R¹)CO- groups.

In general, residues of reactive functional groups represented by X¹ or X² include residues of any groups capable of reacting with any thiol, amino, carboxyl, hydroxyl, aldehyde, aromatic or heteroaromatic group. Thus, for example, X¹ or X² may be -CH₂-, -S-, -NH-, -NHN=, -N(CH₃)N=, -NHCONHN=, -NHCSNHN=, -N(Ph)N= (where Ph is phenyl), -NC(O)-, -NC(S)-, -CO-, 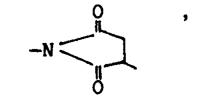
-Het¹C(Het²)CH₂- (where Het¹ and Het², which may be the same or different, is each a nitrogen containing heterocyclic group, e.g. a pyridyl group, or Het¹ is a nitrogen containing heterocyclic group and Het² is a hydrogen atom), or 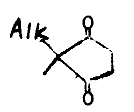
(where Alk is a C₁₋₄ alkyl, e.g. methyl group).

It will be appreciated that when the bridge is branched a reactive functional group may be provided in each branch, thus allowing attachment of the bridge to each chain through more than one bridging site.

The term labelled antibody fragment molecule in conjugates according to the invention is intended to mean an antibody fragment molecule to which a reporter or effector group is attached.

Reporter group is to be understood to mean any group or compound which is easily detectable by analytical means in vitro and/or in vivo and which confers this property to the conjugate. Effector group is to be understood to mean any group or compound which is capable of eliciting a change in, or a response from, a biological system and which also confers this property to the conjugates of the invention.

Suitable reporter or effector molecules include radionuclides, e.g. ¹²⁵I and ¹³¹I; chelated metals; fluorescent compounds or compounds which may be detected by NMR or ESR spectroscopy; pharmacological agents, including cytotoxic compounds and toxins, e.g. ricin and fragments thereof; enzymes; and hormones.

Chelated metals include chelates of di- or tripositive metals having a coordination number from 2 up to 8 inclusive. Particular examples of such metals include technetium (Tc), rhenium (Re), cobalt (Co), copper (Cu), gold (Au), silver (Ag), lead (Pb), bismuth (Bi), indium (In), gallium (Ga), yttrium (Y), terbium (Tb), gadolinium (Gd), and Scandium (Sc). In general the metal is preferably a radionuclide. Particular radionuclides include ^{99m}Tc, ¹⁸⁶Re, ¹⁸⁸Re, ⁵⁸Co, ⁶⁰Co, ⁶⁷Cu, ¹⁹⁵Au, ¹⁹⁹Au, ¹¹⁰Ag, ¹¹¹Ag, ²⁰³Pb, ²⁰⁶Bi, ²⁰⁷Bi, ¹¹¹In, ⁶⁷Ga, ⁶⁸Ga, ⁸⁸Y, ⁹⁰Y, ¹⁶⁰Tb, ¹⁵³Gd and ⁴⁷Sc.

The chelated metal may be for example one of the above types of metal chelated with any suitable polydentate chelating agent, for example acyclic or cyclic polyamines, polyethers, (e.g. crown ethers and derivatives thereof); polyamides; porphyrins; and carbocyclic derivatives.

In general, the type of chelating agent will depend on the metal in use. One particularly useful group of chelating agents in conjugates according to the invention, however, are acyclic and cyclic polyamines, especially polyaminocarboxylic acids, for example diethylenetriaminepentaacetic acid and derivatives thereof, and macrocyclic amines, e.g. cyclic tri-aza and tetra-aza derivatives; and polyamides, especially desferrixoamine and derivatives thereof.

Examples of particular macrocyclic amines include compounds of formula (1) 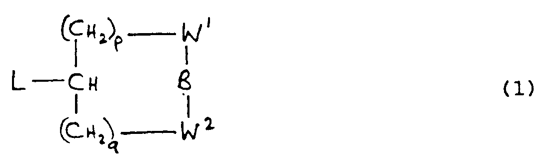
(wherein L is a reactive group, B is a C₂₋₁₄ alkylene chain interrupted by one or two optionally substituted nitrogen atoms; W¹ and W², which may be the same or different, is each an optionally substituted nitrogen atom; p is zero or an integer 1 and q is zero or an integer 1 or 2 with the proviso that when p is zero, q is an integer 1 or 2). It will be appreciated that the group L provides an attachment point for the macrocyle in conjugates according to the invention.
Preferred amines of formula (1) include tri-aza derivatives of formula (2): 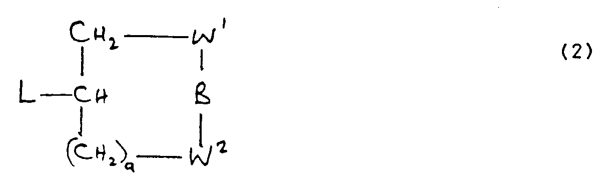
[wherein the group L is as just defined, W¹ and W² which may be the same or different is each a group -N[(CH₂)ᵣR¹]- (where r is zero or an integer 1 to 6 and R¹ is an alkyl, alkoxyalkyl, -CO₂H, -SO₃H, -PO₃H₂ or aryl group) and B is a group -CH₂(CH₂)ₛN(R)(CH₂)ₜCH₂-(where s and t, which may be the same or different is each zero or an integer 1, 2, or 3; and R represents -(CH₂)ᵣR¹ where r and R¹ are as just described)]; and tetra-aza derivatives of formula (3): 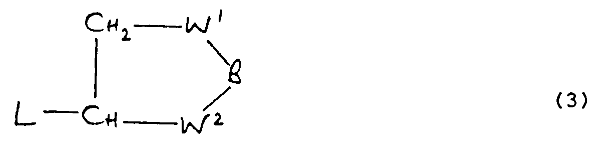
[wherein the group L is as just defined, W¹ and W² which may be the same of different is each a group -N[(CH₂)ᵣR¹]- (as just defined) and B is a group -CH₂(CH₂)ₛN(R)CH₂(CH₂)_{d}N(R)(CH₂)ₜCH₂- (where d is zero or an integer 1, 2, or 3 and s, t and R are as just defined].

A particularly useful amine of formula (2) is the compound of formula (4):

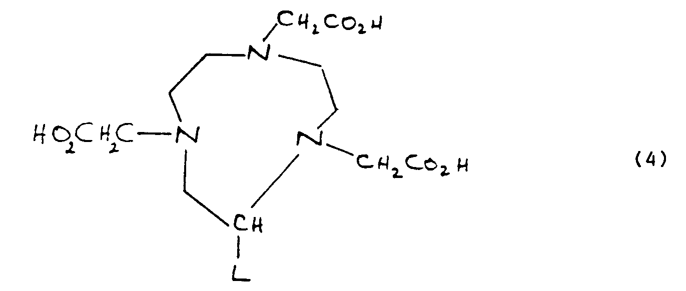

A particularly useful amine of formula (3) is the compound of formula (5): 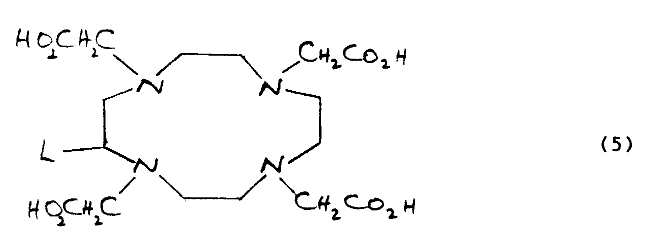
Preferred chelated metals in conjugates according to the invention include indium chelated by a compound of formula (2), particularly the compound of formula (4); or yttrium chelated by a compound of formula (3), particularly the compound of formula (5). ¹¹¹In and ⁹⁰Y are particularly preferred.

The reporter or effector group may be attached to the antibody fragment either directly (for example as in the case of a radionuclide such as iodine) or indirectly through a reactive functional group, for example a group X¹ as described above.

The antibody fragment in the conjugates according to the invention may in general belong to any immunoglobulin class. Thus for example it may be an immunoglobulin M antibody or, in particular, an immunglobulin G antibody, including the isotypes IgG1, IgG2, IgG3 and IgG4. The istoypes IgG1, IgG2 and IgG4 are particularly useful in the conjugates according to the invention, especially the isotypes IgG1 and IgG4.

The antibody fragment molecule may be of animal, for example mammalian origin, and may be for example of murine, rat or human origin. It may be a fragment of a natural antibody, or, if desired, a recombinant antibody fragment i.e. an antibody fragment which has been produced using recombinant DNA techniques.

Particular recombinant antibody fragments include, (1) those having an antigen binding site at least part of which is derived from a different antibody, for example those in which the hypervariable or complementarity determining regions of one antibody have been grafted into the variable framework regions of a second, different antibody (as described in European Patent Specification No. 239400); (2) recombinant fragments wherein non-Fv sequences have been substituted by non-Fv sequences from other, different antibodies (as described in European Patent Specification Nos. 171496, 173494 and 194276); or (3) recombinant fragments possessing substantially the structure of a natural immunoglobulin but wherein one or more amino acid residues is or are altered, for example wherein the hinge region of the antibody fragment has a different number of cysteine residues from that found in the natural immunoglobulin, or wherein one or more cysteine residues in a surface pocket of the recombinant antibody fragment is in the place of another amino acid residue present in the natural immunoglobulin (as described in International Patent Specifications Nos WO89/01974 and WO89/01782 respectively) or wherein a lysine residue is in the place of another amino acid residue present in the natural immunoglobulin.

The antibody fragment may be for example a proteolytic fragment, obtained by enzymatic digestion of a whole antibody. Alternatively, the antibody fragment may be a fragment obtained by recombinant DNA techniques, for example Fv fragments (as described in International Patent Specification No. WO89/02465).

In general, conjugates according to the invention in which the antibody fragment is a F(ab')₂ fragment are preferred.

The antibody fragment may be of polyclonal or, preferably, monoclonal origin. It should be specific for one antigenic determinant. The antigenic determinant may be any hapten or an antigenic determinant associated with any antigen. Particular antigens include those associated with animals, e.g. humans, [for example normal animal tissue or organ cell-associated antigens, tumour cell-associated antigens (for example oncofetal antigens such as carcinoembryonic antigen or alphafetoprotein, placental antigens such as chorionic gonadotropin and placental alkaline phosphatase, and prostate antigens such as prostatic acid phosphatase and prostate specific antigen) and antigens associated with components of body fluids such as fibrin or platelets], viruses, bacteria and fungi.

In a preferred aspect the antibody fragment may be capable of recognising and binding a tumour cell-associated antigen, particularly one or more epitopes on the TAG-72 antigen associated with human breast and colon tumours. A particularly preferred antibody fragment of this type is a fragment of the monoclonal antibody B72.3 [Colcher, D. et al Proc. Nat. Acad. Sci. USA (1981), 78, 3199], particularly a F(ab')₂ fragment.

One preferred group of conjugates according to the invention is that wherein each antibody fragment conjugate comprises a labelled antibody fragment molecule having a non-disulphide inter-heavy chain bridge, said bridge being attached to each heavy chain through the sulphydryl group of a cysteine residue present in each of said chains in the hinge region of said antibody fragment molecule.

Particularly useful conjugates of this type are those in which the antibody fragment has only one cysteine residue present in each heavy chain in the hinge region, said cysteine residues forming the bridging sites for the interchain bridge.

Other useful conjugates of this type are those wherein the antibody fragment is capable of recognising and binding a tumour cell-associated antigen, especially one or more epitopes on the TAG-72 antigen associated with human breast and colon tumours. The antibody fragment may be a fragment of a naturally occurring antibody, particularly a F(ab')₂ fragment, or a recombinant antibody fragment as hereinbefore described. The antibody fragment is preferably a fragment of a B72.3 antibody, including a recombinant B72.3 antibody fragment.

In conjugates of this preferred type the label is preferably an iodine atom, especially ¹²⁵I or ¹²¹I, or a chelating agent of formula (1) particularly be a compound of formulae (2) or (3), especially a compound of formulae (4) or (5) complexed with a metal. The metal is preferably a di- or tripositive metal having a coordination number from 2 up to 8 inclusive and is especially a radionuclide. Indium, especially ¹¹¹In and yttrium, especially ⁹⁰Y are particularly preferred.

The conjugates according to the invention are of use as diagnostic or therapeutic agents. Thus depending on the nature of the antibody fragment and the reporter or effector group, the conjugate may be used in vivo in conjunction with a suitable detector to image normal or diseased tissues, including tumours and thrombi; or in the treatment of abnormal cell disorders, e.g. tumours, thrombi and diseased, including infected, tissues. Alternatively, conjugates according to the invention may be of use in in vitro diagnostic techniques, for example in radioimmunoassays or enzyme-linked immunoassays.

Thus according to a further aspect of the invention we provide an antibody fragment conjugate for use in a method of treatment or diagnosis of a human or animal subject, said antibody fragment conjugate comprising an antibody fragment specific for one antigenic determinant having covalently attached thereto a reporter or effector group characterised in that the antibody fragment conjugate is cross-linked with at least one non-disulphide interchain bridge, said bridge being attached to each chain at one or more bridging sites located outside of the antigen binding domains of the antibody.

For in vivo use the antibody conjugate may be formulated as a suitable composition in an appropriate dosage.

Thus according to another aspect of the invention there is provided a pharmaceutical composition comprising an antibody fragment conjugate according to the present invention; together with one or more pharmaceutically acceptable carriers or excipients.

In vivo administration of the conjugate may be by any suitable route, and is preferably parenteral, e.g. by injection or infusion. Suitable formulations of the conjugate for parenteral administration include suspensions, solutions or emulsions of the conjugate in oily or aqueous vehicles, and may contain formulatory agents such as suspending, stabilising and/or dispersing agents. Alternatively, the conjugate may be in powder form for reconstitution with a suitable vehicle, e.g. sterile pyrogen-free water, before use. If desired, the conjugate may be presented in unit dosage form, and/or together with one or more other active ingredients or imaging agents.

The precise doses at which the conjugate will be administered will depend on the route of administration, nature of the antibody fragment and reporter or effector group and the intended use, i.e. whether for diagnosis or therapy, together with other variables such as the body weight and pathology of the patient. Thus the dose for any application will usually need to be determined empirically, using standard procedures.

Where the conjugate according to the invention is intended for in vitro diagnosis it may be adapted for use employing conventional procedures, for example as described in Methods in Enzymology 84, 1982, and 92, 377-523, 1983 (Gen. Ed. Langone, J.J and Van Vunakis, H, Academic Press, New York).

Conjugates according to the invention may generally be prepared by reaction of an antibody fragment thereof with a cross-linking reagent. It will be appreciated that the cross-linking reaction may be effected with either a labelled or unlabelled antibody fragment molecule.

The reaction may generally be effected in a suitable solvent, e.g. an aqueous solvent such as water or an aqueous inorganic or organic buffer e.g. a phosphate, citrate or acetate buffer or mixtures thereof; or an aqueous organic solvent for example aqueous acetone or dimethylformamide, at any appropriate temperature, for example in the range 0° - 30°C, especially around room temperature.

It will be appreciated that in cross-linking reactions of this general type, where the antibody fragment and/or reporter and effector groups when present contain a number of potentially reactive groups, indiscriminate cross-linking can occur, leading to a heterogeneous mixture of products. To avoid this, the general cross-linking method may be adapted, through appropriate choice of reactants and/or reaction conditions either alone or in combination, to obtain a homgeneous product.

Thus, in one option, cross-linking reagents may be chosen with functional groups that selectively react with specific functional groups in the antibody fragment. There are numerous examples of such groups, for example amino-reactive and thiol-reactive functional groups, well-known to the skilled man, [for example as described in European Patent Specifications Nos. 173629 and 175617, UK Patent Specification No. 2109407 and International Patent Specification No. WO 88/05433] which under appropriate conditions react in a selective manner.

In a second option, potentially reactive groups in the antibody fragment and/or reporter or effector group, which it is not desired to cross-link, may be protected before the cross-linking reaction is effected. Conventional procedures for the protection of reactive groups in proteins may be employed, together with standard deprotection techniques.

In a further option, the antibody fragment may be chosen such that it possesses at least one pair of unique bridging sites, which may be utilised for the cross-linkage. Thus, it is possible to partially reduce an antibody fragment, for example using a mild reducing agent such as β-mercaptoethylamine, such that free sulphydryl groups are generated from the disulphide bridges linking the heavy chains of the antibody fragment, while the remainder of the molecule remains substantially unaffected. The sulphydryl groups may then be used as bridging sites for selective reaction with a thiol specific cross-linking reagent. Alternatively, the antibody may be oxidised, using chemical or enzymatic techniques, e.g. as described in European Patent Specification No. 173629, to generate aldehyde groups in carbohydrate side chains which may then be reacted with a cross-linking reagent as generally described above.

In a further alternative, unique bridging sites may be introduced into an antibody fragment using recombinant DNA techniques, prior to reaction with the cross-linking reagent. Thus, for example, an antibody fragment may be provided wherein the number of cysteine residues in the hinge region of each heavy chain has been reduced to one. This may be conveniently obtained by initally producing in an expression vector an operon which includes a DNA sequence encoding an antibody heavy chain having a hinge region normally associated with the CH1 domain of the antibody fragment molecule.

The operon may be produced by splicing a DNA sequence encoding the CH1 region from an antibody of one class to a DNA sequence encoding the hinge region from an antibody of a different class. Alternatively, it may be produced by cloning the CH1 domain and hinge region from an antibody of one class and altering the number of cysteine residue encoding DNA triplets to one such triplet, by site directed mutagenesis, for example by mutation to alanine-encoding sequences. Transfection of a suitable cell line with the vector and subsequent culturing of the transfected line then produces the desired heavy chain polypeptide.

Since the vector only encodes the heavy chain polypeptide, it will be necessary to arrange for the cell line to produce a complementary light chain. In order to achieve this, one of three alternative strategies may be employed. Thus, in a first alternative, the cell line may be transfected with a second vector, the second vector encoding a complementary light chain-derived polypeptide. Preferably the vectors are identical except in so far as the coding sequences and selectable markers are concerned, so as to ensure as far as possible that each polypeptide chain is equally expressed.

In a second alternative, the vector may include sequences coding for both light chain and heavy chain-derived polypeptides. In a third alternative, a host cell which naturally secretes a complementary light chain may be used. The general methods by which the vectors may be constructed, transfection methods and culture methods are well known (see for example Maniatis et al, Molecular Cloning, Cold Spring Harbor, New York, 1982; Primrose and Old, Principles of Gene Manipulation, Blackwell, Oxford 1980). The above method is more particularly described in International Patent Specification No. WO89/01974.

It will be appreciated that the above-described techniques may be adapted to yield any size heavy chain - light chain pair containing a hinge region with one cysteine residue. Such constructs may be reacted with a cross-linking reagent as generally described above and in the Examples below.

Using similar recombinant DNA techniques to those just described (see also International Patent Specification No. WO89/01782) a cysteine residue may be introduced into each heavy chain of an antibody molecule to provide unique bridging sites for subsequent reaction with a cross-linking reagent to produce a conjugate according to the invention. The methods described may also be used in suitably adapted form with approriate starting materials where it is desired to produce other recombinant antibody fragments, for example recombinant antibody fragments containing additional lysine groups or other amino acids which provide unique bridging sites, for the cross-linking reagent.

Generally, we have found that in cross-linking reactions to produce compounds according to the invention, particularly where the antibody fragment has unique bridging sites, for example as described above, it is preferable to react the cross-linking reagent with excess of antibody fragment. By doing this, indiscriminate cross-linking is avoided and the desired antibody fragment product is produced in good yield, and purity. Cross-linking reactions in which the antibody fragment is used in excess concentration (for example 2X and greater excess) relative to the cross-linking reagent form a further feature of the invention.

Where in the conjugates of the invention the reporter or effector group is a chelated metal, the last step in the preparation of the conjugate may be a reaction to introduce the metal. The metal may be introduced by reacting an antibody with a metal salt (for example a metal halide) in an appropriate solvent, for example an aqueous or non aqueous solvent, (e.g. acetonitrile, acetone, propylene carbonate, dimethylformamaide or dimethylsulphoxide) at any suitable temperature from 0°C to 50°C, e.g. around room temperature.

Antibodies for use as starting materials in the preparation of conjugates according to the invention may be obtained by conventional means, for example from the sera of immunised animals, or preferably, myeloma or hybridoma cells, or by recombinant DNA techniques as described in European Patent Specifications 171496, 173494, 194276 and 239400 and International Patent Specifications Nos. WO89/01974, WO89/01782, WO89/02465 and WO89/01783. Antibody fragments may be prepared from whole antibodies by enzymatic or chemical means or a combination of both in accordance with conventional practice, or by the aforementioned recombinant DNA techniques.

Cross-linking reagents for the preparation of conjugates according to the invention may in general be a heterofunctional cross-linking reagent. Methods for obtaining heterofunctional cross-linking reagents are well-known [see for example Ghose, T. I. et al in Methods in Enzymology (1983), 93, 280-333].

Effector or reporter groups for use in the conjugates according to the invention are readily available, (for example see UK Patent Specification 2109407, US Patent Specification 4472509, European Patent Specification No. 175617 and International Patent Specifcations Nos. WO89/01475 and WO89/01476) or may be obtained from these known compounds by simple chemical modification using conventional techniques, or may be prepared from known starting materials using methods analogous to those used for the preparation of the known compounds.

Particular macrocyclic amines of formulae (1) (2) and (3) may be prepared by the methods described in International Patent Specifications Nos. WO89/01475 and WO89/01476.

Reporter or effector groups may be linked to the antibody fragment using conventional means. Thus, for example, iodine may be directly linked to the antibody using the chloramine T method (see for example US Patent Specification 4331647), while other reporter or effector groups may be linked by reaction of suitable functional groups on the reporter or effector group with the antibody fragment using standard procedures, for example as described in UK Patent Specification No. 2109407 and International Patent Specifications Nos. WO89/01475 and WO89/01476.

### Description of Specific Embodiments

The following Examples illustrate the invention.

### Example 1

(a) Preparation of F(ab')₂ fragments from B72.3 IgG [Colcher, D et al Proc. Nat. Acad. Sci. USA (1981), 78, 3199]. F(ab')₂ fragments were prepared from B72.3 IgG by digestion with bromelain. The bromelain was first activated by incubating a 1.0mg/ml solution with 50mM cysteine in 0.1M acetate pH 5.50, containing 3mM EDTA at 37°C for 30 minutes. Activated bromelain was deslated into 0.1M acetate pH5.50, containing 3mM EDTA, using a PD10(Sephadex G25) column, to remove cysteine and added to a 2/0mg.ml solution of B72.3 (1(w.w):50(w/w) enzyme:IgG) in the same buffer. The digest was incubated at 37°C for about 2 hours and conversion from Ig G to a F(ab')₂ sized peak monitored by SDS-PAGE. After competion of the digestion, antipain and leupeptin were added (final concentration 0.05mg/ml) and the pH of the mixture adjusted to pH6.0 with NaOH for subsequent S Sepharose Fast Flow ion-exchange chromatography. The F(ab')₂, Fc and remaining IgG eluted in the flow through and the bromelain remained bound to the matrix. This negative purification step effectively removed all the bromelain from the digest mixture.
(b) Purification of F(ab′)₂ fragements.
   The remaining digest mixture was first dialysed into 20mM Tris pH7.8 and loaded onto a DEAE Sepharose Fast Flow column. The F(ab′)₂ eluted from the column in the flow through whilst the Fc portion and remaining intact IgG were retained. The bound material was eluted from the matrix with 0.1M NaCl in 20mM Tris pH7.8 to regenerate the column.
(c) Preparation of chemically cross-linked F(ab′)₂ molecules.
   B72.3 F(ab′)₂ at 5.0mg/ml in 0.15M phosphate buffer pH8.0, containing 2mM EDTA was reduced by the addition of 2-mecrcaptoethylamine to a final concentration of 5mM and incubation at 37^{o}C for 30 minutes. After reduction, the sample was desalted down a Sephadex G25 (Pharmacia) column equilibrated with 0.1M citric acid/0.2M Na₂HPO₄ pH6.0 containing 2mM EDTA.
   1,6-Bismaleimidohexane was dissolved in dimethylformamide to a final concentration of 72mM and added to the feshly reduced Fab′SH at a level of 0.9mM (approximatley 22 fold excess over titratable thiols). After 90 minutes incubation with constant mixing at room temperature N-ethyl maleimide was added to a final concentration of 9mM and incubated further for 30 minutes before desalting into 0.1M citric acid/0.2M Na2HPO4 2mM EDTA. The maleimidated Fab′ (Fab′mal) was immediately added to a fresh batch of Fab′SH at a molar ratio of Fab′mal:Fab′SH of 1.1:1.0 and incubated at room temperature with constant mixing for about 20 hours. The cross-linked F(ab′)₂ was purified from the reaction mixture by HPLC gel filtration.
   The composition of the cross-linking reaction mixture was determined by HPLC gel filtration after the overnight incubation. 10»l of the reaction mixture was added to 10»l of 200mM 2-mercaptoethylamine and incubated for 15 minutes. A further addition of 20»l of 800mM iodoacetamide was made and reacted for 15 minutes. The reduced and alkylated sample was then analysed by HPLC GF 250 and the percentage composition of the chromatogram determined by integration. The chemically cross-linked molecule eluted with a retention time of 8.62 min whereas unreacted Fab′ eluted later at 9.48 min under standard HPLC conditions of 1ml/min flow rate in 0.2M phosphate buffer pH7.0 mobile phase. The cross-linked F(ab′)₂ was also verified after purification by reducing SDS-PAGE where the F(ab′)₂ showed a cross-linked H′-H′ band (Mr:∼46,000) and L band (Mr∼22,000) whereas a control showed the usual H′(Mr:23,000) and L bands (Mr:22,000).

The reaction was repeated using the following cross-linking reagents in place of 1,6-bismaleimidohexane: N,N′-O-phenylene dimaleimide; bismaleimido methyl ether; and lysine bismaleimide.

Each of the cross-linked antibodies prepared above was iodinated using the chloramine T method to yield a conjugate antibody of the invention wherein the label is iodine and the bridging group is either the residue of 1, 6 bismaleimidohexane, N,N¹-O-phenylene dimaleimide, bismaleimide methyl ether or lysine bismaleimide linked to each heavy chain of the antibody through a cysteine bridging site present in the hinge region.

### Example 2

This illustrates the preparation of chimeric B72.3 Fab′ delta cys cross-linked with either 1,6-bismaleimidohexane or lysine bismaleimide. The chimeric B72.3 Fab′ delta cys starting material was prepared according to the methods specifically described in International Patent Specifications WO89/01974 and WO89/01783.

Chimeric B72.3 Fab′ delta cys at 1.0 to 2.0 mg/ml in 0.1M sodium acetate buffer pH6.0, containing 0.86mM citric acid and 2mM EDTA was reduced by the addition of 2-mercaptoethylamine to a final concentration of 5mM and incubated at 37^{o}C for 30 minutes. After reduction, samples were desalted down a Sephadex G25 (Pharmacia) column, equilibrated with 0.1M sodium acetate/citrate buffer, pH6.0 containing 2mM EDTA.

Lysine bismaleimide was dissolved in water and added to the freshly reduced Fab′SH at a concentration of 3.8mM (approximately 40 fold excess over titratable thiols) and incubated at room temperature for 60 minutes with constant mixing. N-ethyl maleimide was added to a final concentration of 9mM and incubated further for 30 minutes before desalting into phosphate buffered saline pH7.5. The malemidated Fab′ (Fab′mal) was immediately added to a fresh batch of Fab′SH at a weight ratio of Fab′mal:Fab′SH of 1.1:1,0 and incubated at room temperature with constant mixing for about 20 hours.

The composition of the cross linking reaction mixture was determined by HPLC gel filtration after the overnight incubation. 10»l of the reaction mixture was added to 10»l of 200mM 2-mercaptoethylamine and incubated for 15 minutes. A further addition of 20»l of 800mM iodoacetamide was made and reacted for 15 minutes. The reduced alkylated sample was then analysed using HPLC (Zorbax GF-250 gel filtration column) and the percentage composition of the chromatogram determined by integration. Material cross-linked with lysine bismaleimide eluted with a retention time of 8.62 min whereas unreacted cFab′ eluted later at 9.48 min under standard HPLC conditions of 1ml/min flow rate in 0.2M phosphate buffer pH7.0 mobile phase. The cross-linked material was also verified after purification by reducing SDS-PAGE where it showed a cross-linked H′-H′ band (Mr:∼46,000) and L band (Mr:∼22,000) whereas a control showed the usual Hl (MR;23,000) and L bands (Mr:22,000).

The experiment was repeated using 1,6-bismaleimidohexane in dry dimethylformamide in place of the lysine bismaleimide in water to yield antibody material cross-linked with bismaleimidohexane.

The products from both experiments were iodinated using the chloramine T method to yield a conjugate antibody of the invention wherein the label is iodoine and the bridging group is either the residue of lysine bismaleimide or 1,6 bismaleimidohexane, linked to each heavy chain of the antibody through the single cysteine residue present in each chain.

### Example 3

The experiments described in Example 2 were repeated except that each cross-linker was added to the reduced Fab′SH at a molar equivalent ratio of Fab′SH: cross-linker of 2.2:1. After incubation at 37^{o}C for 60 minutes, cross-linked material was isolated as described in Example 2 and shown by SDS-PAGE to be identical to the material obtained in Example 2. Subsequent iodination as described in Example 2 yield labelled cross-linked antibody.

## Claims (Claims for the following Contracting State(s): AT, BE, CH, DE, DK, FR, GB, IT, LI, LU, NL, SE)

1. An antibody fragment conjugate comprising an antibody fragment specific for one antigenic determinant having covalently attached thereto a reporter or effector group characterised in that the antibody fragment conjugate is cross-linked with at least one non-disulphide interchain bridge, said bridge being attached to each chain at one or more bridging sites located outside of the antigen binding domains of the antibody.

2. A conjugate according to Claim 1 wherein said bridging site is an amino, sulphydryl, carboxyl, phenolic or other aromatic or heteroaromatic functional group present in the side-chain of an amino acid residue.

3. A conjugate according to Claim 2 wherein said bridging site is a sulphydryl group present in the side-chain of a cysteine residue.

4. A conjugate according to Claim 3 wherein the cysteine residue is present in each heavy chain in the hinge region.

5. A conjugate according to Claim 4 wherein the cysteine residue is the only cysteine residue present in the hinge region.

6. A conjugate according to any of the preceding claims wherein the antibody is a recombinant antibody fragment.

7. A conjugate according to any one of the preceding claims wherein the fragment is a F(ab')₂ fragment.

8. A conjugate according to any of the preceding claims wherein the non-disulphide interchain bridge is the residue of a homo- or heterofunctional cross-linking reagent.

9. A conjugate according to any one of the preceding claims wherein the reporter or effector group is a radionuclide, a chelated metal, a fluorescent compound, a compound which may be detected by NMR or ESR spectroscopy, a pharmacological agent, an enzyme or a hormone.

10. A conjugate according to Claim 9 wherein the reporter or effector group is a radionuclide or chelated metal.

11. A conjugate according to Claim 10 wherein the radionuclide is radioiodide.

12. A conjugate according to Claim 11 wherein the chelated metal is a chelate of a di- or tripositive metal having a co-ordination number from 2 up to 8 inclusive and a polydentate chelating agent.

13. A conjugate according to Claim 12 wherein the polydentate chelating agent is an acyclic or cyclic polyamine, a polyether, a polyamide, a porphyrin or a carbocyclic derivative.

14. A conjugate according to Claim 13 wherein the acyclic polyamine is a polyaminocarboxylic acid.

15. A conjugate according to Claim 13 wherein the cyclic polyamine is a cyclic tri-aza or tetra-aza derivative.

16. A pharmaceutical composition comprising a antibody fragment conjugate as defined in any one of the preceding claims in association with one or more pharmaceutically acceptable carriers or excipients.

17. A process for the preparation of an antibody fragment conjugate as defined in any one of claims 1 to 15 which comprises reacting a cross-linking reagent with an antibody fragment.

18. A process according to Claim 19 wherein the antibody fragment is present in excess concentration relative to the cross-linking reagent.

19. An antibody fragment as defined in any one of claims 1 to 15 for use in a method of treatment or diagnosis of a human or animal subject.

## Claims (Claims for the following Contracting State(s): ES, GR)

1. A method for the preparation of an antibody fragment conjugate comprising an antibody fragment specific for one antigenic determinant having covalently attached thereto a reporter or effector group wherein the antibody fragment conjugate is cross-linked with at least one non-disulphide interchain bridge, said bridge being attached to each chain at one or more bridging sites located outside of the antigen binding domains of the antibody, wherein the method comprises reacting a cross-linking reagent with an antibody fragment.

2. A method according to claim 1 wherein the cross-linking reagent and antibody fragment are reacted in a solvent.

3. A method according to claim 2 wherein the solvent is an aqueous solvent or an aqueous organic solvent.

4. A method according to any preceding claim wherein the cross-linking reagent and antibody fragment are reacted at a temperature in the range 0°-30°C.

5. A method according to any preceding claim wherein said bridging site is an amino, sulphydryl, carboxyl, phenolic or other aromatic or heteroaromatic functional group present in the side-chain of an amino acid residue.

6. A method according to claim 5 wherein said bridging site is a sulphydryl group present in the side-chain of a cysteine residue.

7. A method according to claim 6 wherein the cysteine residue is present in each heavy chain in the hinge region.

8. A method according to claim 7 wherein the cysteine residue is the only cysteine residue present in the hinge region.

9. A method according to any of the preceding claims wherein the antibody fragment is a recombinant antibody fragment.

10. A method according to any of the preceding claims wherein the antibody fragment is a F(ab')₂ fragment.

11. A method according to any of the preceding claims wherein the non-disulphide interchain bridge is the residue of a homo- or heterofunctional cross-linking reagent.

12. A method according to any of the preceding claims wherein the reporter or effector group is a radionuclide, a chelated metal, a fluorescent compound, a compound which may be detected by NMR or ESR spectroscopy, a pharmacological agent, an enzyme or a hormone.

13. A method according to claim 12 wherein the reporter or effector group is a radionuclide or chelated metal.

14. A method according to claim 13 wherein the radionuclide is radioiodide.

15. A method according to claim 13 wherein the reporter or effector group comprises a chelated metal, the method comprising the further step of reacting the cross-linked antibody fragment conjugate with a metal salt in order to introduce the metal.

16. A method according to claim 13 or claim 15 wherein the chelated metal is a chelate of a di- or tripositive metal having a co-ordination number from 2 up to 8 inclusive and a polydentate chelating agent.

17. A method according to claim 16 wherein the polydentate chelating agent is an acyclic or cyclic polyamine, a polyether, a polyamide, a porphyrin or a carbocyclic derivative.

18. A method according to claim 17 wherein the acyclic polyamine is a polyaminocarboxylic acid.

19. A method according to claim 17 wherein the cyclic polyamine is a cyclic tri-aza or tetra-aza derivative.

20. A method according to any preceding claim wherein the antibody fragment is present in excess concentration relative to the cross-linking reagent.

21. A method for the manufacture of a pharmaceutical composition comprising a antibody fragment conjugate as defined in any one of the preceding claims, wherein the method comprises: bringing into association a pharmaceutically effective amount of said antibody fragment conjugate with a pharmaceutically acceptable carrier, excipient or diluent.

## Patentansprüche (Patentansprüche für folgende(n) Vertragsstaat(en): AT, BE, CH, DE, DK, FR, GB, IT, LI, LU, NL, SE)

1. Antikörperfragment-Konjugat, umfassend ein für eine antigene Determinate spezifisches Antikörperfragment, an welchem eine Reporter- oder Effektorgruppe kovalent angebracht ist, dadurch gekennzeichnet, daß das Antikörperfragment-Konjugat mit mindestens einer Nicht-Disulfid-Zwischenkettenbrücke vernetzt ist, wobei die Brücke an jede Kette an einer oder mehreren Brückenstellen, die außerhalb der Antigen-bindenden Bereiche des Antikörpers angeordnet sind, angebracht ist.

2. Konjugat nach Anspruch 1, wobei die Brückenstelle eine in der Seitenkette eines Aminosäurerestes vorhandene Amino-, Sulfhydryl-, Carboxyl- oder Phenolgruppe oder eine andere aromatische oder heteroaromatische funktionelle Gruppe ist.

3. Konjugat nach Anspruch 2, wobei die Brückenstelle eine in der Seitenkette eines Cysteinrestes vorhandene Sulfhydrylgruppe ist.

4. Konjugat nach Anspruch 3, wobei der Cysteinrest in jeder schweren Kette im Gelenksbereich vorhanden ist.

5. Konjugat nach Anspruch 4, wobei der Cysteinrest der einzige, im Gelenksbereich vorhandene Cysteinrest ist.

6. Konjugat nach einem der vorhergehenden Ansprüche, wobei der Antikörper ein rekombinantes Antikörperfragment ist.

7. Konjugat nach einem der vorhergehenden Ansprüche, wobei das Fragment ein F(ab')₂-Fragment ist.

8. Konjugat nach einem der vorhergehenden Ansprüche, wobei die Nicht-Disulfid-Zwischenkettenbrücke der Rest eines homo- oder heterofunktionellen Vernetzungsmittels ist.

9. Konjugat nach einem der vorhergehenden Ansprüche, wobei die Reporter- oder Effektorgruppe ein Radionuklid, ein chelatisiertes Metall, eine fluoreszierende Verbindung, eine Verbindung, welche mittels NMR- oder ESR-Spektroskopie nachgewiesen werden kann, ein pharmakologisches Mittel, ein Enzym oder ein Hormon ist.

10. Konjugat nach Anspruch 9, wobei die Reporter- oder Effektorgruppe ein Radionuklid oder ein chelatisiertes Metall ist.

11. Konjugat nach Anspruch 10, wobei das Radionuklid Radiojodid ist.

12. Konjugat nach Anspruch 11, wobei das chelatisierte Metall ein Chelat eines zwei- oder dreipositiven Metalls mit einer Koordinationszahl von 2 bis einschließlich 8 und eines vielzähnigen Chelatbildners ist.

13. Konjugat nach Anspruch 12, wobei der vielzähnige Chelatbildner ein acyclisches oder cyclisches Polyamin, ein Polyether, ein Polyamid, ein Porphyrin oder ein carbocyclisches Derivat ist.

14. Konjugat nach Anspruch 13, wobei das acyclische Polyamin eine Polyaminocarbonsäure ist.

15. Konjugat nach Anspruch 13, wobei das cyclische Polyamin ein cyclisches Triaza- oder Tetrazaderivat ist.

16. Pharmazeutische Zusammensetzung, umfassend ein Antikörperfragment-Konjugat, wie in einem der vorhergehenden Ansprüche beansprucht, in Verbindung mit einem oder mehereren pharmazeutischen Trägern oder Exzipienten.

17. Verfahren zur Herstellung eines Antikörperfragment-Konjugates, wie in einem der Ansprüche 1 bis 15 beansprucht, welches ein Umsetzen eines Vernetzungsmittels mit einem Antikörperfragment umfaßt.

18. Verfahren nach Anspruch 19, wobei das Antikörperfragment in Überschußkonzentration relativ zum Vernetzungsmittel vorhanden ist.

19. Antikörperfragment, wie in einem der Ansprüche 1 bis 15 definiert, zur Verwendung in einem Verfahren zur Behandlung oder Diagnose an einein Menschen oder einem Tier.

## Patentansprüche (Patentansprüche für folgende(n) Vertragsstaat(en): ES, GR)

1. Verfahren zur Herstellung eines Antikörperfragment-Konjugates, das ein für eine antigene Determinate spezifisches Antikörperfragment umfaßt, an welchem eine Reporter- oder Effektorgruppe kovalent angebracht ist, wobei das Antikörperfragment-konjugat mit mindestens einer Nicht-Disulfid-Zwischenkettenbrücke vernetzt ist, welche Brücke an jede Kette an einer oder mehreren Brückenstellen, die außerhalb der Antigen-bindenden Bereiche des Antikörpers angeordnet sind, angebracht ist, wobei das Verfahren ein Umsetzen eines Vernetzungsmittels mit einem Antikörperfragment umfaßt.

2. Verfahren nach Anspruch 1, wobei das Vernetzungsmittel und das Antikörperfragment in einem Lösungsmittel umgesetzt werden.

3. Verfahren nach Anspruch 2, wobei das Lösungsmittel ein wäßriges Lösungsmittel oder ein wäßriges organisches Lösungsmittel ist.

4. Verfahren nach einem vorhergehenden Anspruch, wobei das Vernetzungsmittel und das Antikörperfragment bei einer Temperatur im Bereich von 0°-30°C umgesetzt werden.

5. Verfahren nach einem vorhergehenden Anspruch, wobei die Brückenstelle eine in der Seitenkette eines Aminosäurerestes vorhandene Amino-, Sulfhydryl-, Carboxyl- oder Phenolgruppe oder eine andere aromatische oder heteroaromatische funktionelle Gruppe ist.

6. Verfahren nach Anspruch 5, wobei die Brückenstelle eine in der Seitenkette eines Cysteinrestes vorhandene Sulfhydrylgruppe ist.

7. Verfahren nach Anspruch 6, wobei der Cysteinrest in jeder schweren Kette im Gelenksbereich vorhanden ist.

8. Verfahren nach Anspruch 7, wobei der Cysteinrest der einzige, im Gelenksbereich vorhandene Cysteinrest ist.

9. Verfahren nach einem der vorhergehenden Ansprüche, wobei das Antikörperfragment ein rekombinantes Antikörperfragment ist.

10. Verfahren nach einem der vorhergehenden Ansprüche, wobei das Antikörperfragment ein F(ab')₂-Fragment ist.

11. Verfahren gemäß einem der vorhergehenden Ansprüche, wobei die Nicht-Disulfid-Zwischenkettenbrücke der Rest eines homo- oder heterofunktionellen Vernetzungsmittels ist.

12. Verfahren nach einem der vorhergehenden Ansprüche, wobei die Reporter- oder Effektorgruppe ein Radionuklid, ein chelatisiertes Metall, eine fluoreszierende Verbindung, eine Verbindung, welche mittels NMR- oder ESR-Spektroskopie nachgewiesen werden kann, ein pharmakologisches Mittel, ein Enzym oder ein Hormon ist.

13. Verfahren nach Anspruch 12, wobei die Reporter- oder Effektorgruppe ein Radionuklid oder ein chelatisiertes Metall ist.

14. Verfahren nach Anspruch 13, wobei das Radionuklid Radiojodid ist.

15. Verfahren nach Anspruch 13, wobei die Reporter- oder Effektorgruppe ein chelatisiertes Metall umfaßt und das Verfahren den weiteren Schritt eines Umsetzens des vernetzten Antikörperfragment-Konjugates mit einem Metallsalz umfaßt, um das Metall einzubringen.

16. Verfahren nach Anspruch 13 oder Anspruch 15, wobei das chelatisierte Metall ein Chelat eines zwei- oder dreipositiven Metalls mit einer Koordinationszahl von 2 bis einschließlich 8 und eines vielzähnigen Chelatbildners ist.

17. Verfahren nach Anspruch 16, wobei der vielzähnige Chelatbildner ein acyclisches oder cyclisches Polyamin, ein Polyether, ein Polyamid, ein Porphyrin oder ein carbocyclisches Derivat ist.

18. Verfahren nach Anspruch 17, wobei das acyclische Polyamin eine Polyaminocarbonsäure ist.

19. Verfahren nach Anspruch 17, wobei das cyclische Polyamin ein cyclisches Triaza- oder Tetrazaderivat ist.

20. Verfahren nach einem vorhergehenden Anspruch , wobei das Antikörperfragment in Überschußkonzentration relativ zum Vernetzungsmittel vorhanden ist.

21. Verfahren zur Herstellung einer pharmazeutischen Zusammensetzung, welche ein Antikörperfragment-Konjugat, wie in einem der vorhergehenden Ansprüche beansprucht, umfaßt, wobei das Verfahren umfaßt, daß eine pharmazeutisch wirksame Menge des Antikörperfragment-Konjugates mit einem pharmazeutisch akzeptablen Träger, Exzipienten, oder Verdünnungsmittel in Verbindung gebracht wird.

## Revendications (Revendications pour l'(les) Etat(s) contractant(s) suivant(s): AT, BE, CH, DE, DK, FR, GB, IT, LI, LU, NL, SE)

1. Conjugué de fragment d'anticorps comprenant un fragment d'anticorps spécifique d'un déterminant antigénique ayant un groupe marqueur ou effecteur lié à lui de façon covalente, caractérisé en ce que le conjugué de fragment d'anticorps est réticulé avec au moins un pont interchaîne non-disulphure, ledit pont étant relié à chaque chaîne sur un ou plusieurs sites de pontage situés à l'extérieur des domaines de fixation de l'antigène de l'anticorps.

2. Conjugué selon la revendication 1, dans lequel ledit site de pontage est un groupe fonctionnel amino, sulfhydryle, carboxyle, phénolique ou autre groupe fonctionnel aromatique ou hétéroaromatique présent dans la chaîne latérale d'un résidu d'aminoacide.

3. Conjugué selon la revendication 2, dans lequel ledit site de pontage est un groupe sulfhydryle présent dans la chaîne latérale d'un résidu de cystéine.

4. Conjugué selon la revendication 3, dans lequel le résidu de cystéine est présent dans chaque chaîne lourde dans la région charnière.

5. Conjugué selon la revendication 4, dans lequel le résidu de cystéine est le seul résidu de cystéine présent dans la région charnière.

6. Conjugué selon l'une quelconque des revendications précédentes, dans lequel l'anticorps est un fragment d'anticorps recombinant.

7. Conjugué selon l'une quelconque des revendications précédentes, dans lequel le fragment est un fragment F(ab')₂.

8. Conjugué selon l'une quelconque des revendications précédentes, dans lequel le pont interchaîne non-disulphure est le résidu d'un réactif réticulant homo- ou hétérofonctionnel.

9. Conjugué selon l'une quelconque des revendications précédentes, dans lequel le groupe marqueur ou effecteur est un radionucléide, un métal chélaté, un composé fluorescent, un composé qui peut être détecté par une spectroscopie RMN ou ESR, un agent pharmacologique, une enzyme ou une hormone.

10. Conjugué selon la revendication 9, dans lequel le groupe marqueur ou effecteur est un radionucléide ou un métal chélaté.

11. Conjugué selon la revendication 10, dans lequel le radionucléide est une iodure radioactif.

12. Conjugué selon la revendication 11, dans lequel le métal chélaté est un chélate d'un métal di- ou tripositif ayant une coordinence comprise entre 2 et 8 inclus et un agent chélatant polydentate.

13. Conjugué selon la revendication 12, dans lequel l'agent chélatant polydentate est une polyamine acyclique ou cyclique, un polyéther, un polyamide, une porphyrine ou un dérivé carbocyclique.

14. Conjugué selon la revendication 13, dans lequel la polyamine acyclique est un acide polyaminocarboxylique.

15. Conjugué selon la revendication 13, dans lequel la polyamine cyclique est un tri- ou tétra-azadérivé cyclique.

16. Composition pharmaceutique comprenant un conjugué de fragment d'anticorps selon l'une quelconque des revendications précédentes associé à un ou plusieurs véhicules ou excipients pharmaceutiquement acceptables.

17. Procédé de préparation d'un conjugué de fragment d'anticorps selon l'une quelconque des revendications 1 à 15, consistant à mettre en réaction un réactif réticulant avec un fragment d'anticorps.

18. Procédé selon la revendication 19, dans lequel le fragment d'anticorps est présent à une concentration excessive par rapport au réactif réticulant.

19. Fragment d'anticorps selon l'une quelconque des revendications 1 à 15 destiné à être utilisé dans une méthode de traitement ou de diagnostic d'un sujet d'espèce humaine ou animale.

## Revendications (Revendications pour l'(les) Etat(s) contractant(s) suivant(s): ES, GR)

1. Procédé de préparation d'un conjugué de fragment d'anticorps comprenant un fragment d'anticorps spécifique d'un déterminant antigénique ayant un groupe marqueur ou effecteur lié à lui de façon covalente, dans lequel le conjugué de fragment d'anticorps est réticulé avec au moins un pont interchaîne non-disulphure, ledit pont étant relié à chaque chaîne sur un ou plusieurs sites de pontage situés à l'extérieur des domaines de fixation de l'antigène de l'anticorps, dans lequel le procédé consiste à mettre en réaction un réactif réticulant avec le fragment d'anticorps.

2. Procédé selon la revendication 1, dans lequel le réactif réticulant et le fragment d'anticorps sont mis en réaction dans un solvant.

3. Procédé selon la revendication 2, dans lequel le solvant est un solvant aqueux ou un solvant organique aqueux.

4. Procédé selon l'une quelconque des revendications précédentes, dans lequel le réactif réticulant et le fragment d'anticorps sont mis en réaction à une température dans la gamme 0°-30°C.

5. Procédé selon l'une quelconque des revendications précédentes, dans lequel ledit site de pontage est un groupe fonctionnel amino, sulfhydryle, carboxyle, phénolique ou autre groupe fonctionnel aromatique ou hétéroaromatique présent dans la chaîne latérale d'un résidu d'aminoacide.

6. Procédé selon la revendication 5, dans lequel ledit site de pontage est un groupe sulfhydryle présent dans la chaîne latérale d'un résidu de cystéine.

7. Procédé selon la revendication 6, dans lequel le résidu de cystéine est présent dans chaque chaîne lourde dans la région charnière.

8. Procédé selon la revendication 7, dans lequel le résidu de cystéine est le seul résidu de cystéine présent dans la région charnière.

9. Procédé selon l'une quelconque des revendications précédentes, dans lequel le fragment d'anticorps est un fragment d'anticorps recombinant.

10. Procédé selon l'une quelconque des revendications précédentes, dans lequel le fragment d'anticorps est un fragment F(ab')₂.

11. Procédé selon l'une quelconque des revendications précédentes, dans lequel le pont interchaîne non-disulphure est le résidu d'un réactif réticulant homo- ou hétérofonctionnel.

12. Procédé selon l'une quelconque des revendications précédentes, dans lequel le groupe marqueur ou effecteur est un radionucléide, un métal chélaté, un composé fluorescent, un composé qui peut être détecté par une spectroscopie RMN ou ESR, un agent pharmacologique, une enzyme ou une hormone.

13. Procédé selon la revendication 12, dans lequel le groupe marqueur ou effecteur est un radionucléide ou un métal chélaté.

14. Procédé selon la revendication 13, dans lequel le radionucléide est un iodure radioactif.

15. Procédé selon la revendication 13, dans lequel le groupe marqueur ou effecteur comprend un métal chélaté, le procédé comportant une étape supplémentaire qui consiste à mettre en réaction le conjugué de fragment d'anticorps réticulé avec un sel métallique afin d'introduire le métal.

16. Procédé selon la revendication 13 ou la revendication 15, dans lequel le métal chélaté est un chélate d'un métal di- ou tripositif ayant une coordinence comprise entre 2 et 8 inclus et un agent chélatant polydentate.

17. Procédé selon la revendication 16, dans lequel l'agent chélatant polydentate est une polyamine acyclique ou cyclique, un polyéther, un polyamide, une porphyrine ou un dérivé carbocyclique.

18. Procédé selon la revendication 17, dans lequel la polyamine acyclique est un acide polyaminocarboxylique.

19. Procédé selon la revendication 17, dans lequel la polyamine cyclique est un tri- ou tétra-azadérivé cyclique.

20. Procédé selon l'une quelconque des revendications précédentes, dans lequel le fragment d'anticorps est présent à une concentration excessive par rapport au réactif réticulant.

21. Procédé de fabrication d'une composition pharmaceutique se composant d'un conjugué de fragment d'anticorps selon l'une quelconque des revendications précédentes, dans lequel le procédé consiste à : provoquer l'association d'une quantité pharmaceutiquement efficace dudit conjugué de fragment d'anticorps avec un véhicule, un excipient ou un diluant pharmaceutiquement acceptable.
